Europäisches Patentamt

European Patent Office

Office européen des brevets

(19)

(11) Numéro de publication: **0 270 979 B1**

(12) **FASCICULE DE BREVET EUROPEEN**

(45) Date de publication de fascicule du brevet:
**15.05.91**

(51) Int. Cl.⁵: **A61F 13/15**

(21) Numéro de dépôt: **87117733.3**

(22) Date de dépôt: **01.12.87**

(54) **Couche-culotte à élastiques longitudinaux, et procédé de fabrication en continu de telles couches-culottes.**

(30) Priorité: **02.12.86 FR 8616844**

(43) Date de publication de la demande:
**15.06.88 Bulletin 88/24**

(45) Mention de la délivrance du brevet:
**15.05.91 Bulletin 91/20**

(84) Etats contractants désignés:
**AT BE CH DE ES FR GB GR IT LI LU NL SE**

(56) Documents cités:
**EP-A- 0 113 976**
**EP-A- 0 158 490**
**DE-A- 3 319 043**
**US-A- 4 353 762**
**US-A- 4 405 397**

(73) Titulaire: **PEAUDOUCE**
**59, Rue de la Vignette**
**F-59126 Linselles(FR)**

(72) Inventeur: **Villez, Yves**
**13, Rue Albert Camus**
**F-59126 Linselles(FR)**

(74) Mandataire: **Casalonga, Axel et al**
**BUREAU D.A. CASALONGA - JOSSE Morassistrasse 8**
**W-8000 München 5(DE)**

## Description

La présente invention se rapport à une couche-culotte du type comprenant une feuille extérieure souple, imperméable aux liquides, dont la face interne est munie de lignes longitudinales de colle, un coussin absorbant disposé sur la face interne de la feuille extérieure de manière que ses deux bords longitudinaux opposés soit en retrait par rapport aux deux bords longitudinaux opposés de la feuille extérieure et que ses deux bords transversaux opposés soient en retrait par rapport aux deux bords transversaux opposés de la feuille extérieure, une feuille intérieure souple, perméable aux liquides, recouvrant la face interne de la feuille extérieure et le coussin absorbant disposé sur cette face, des éléments élastiques longitudinaux fixés par collage à l'état tendu sur la face interne de la feuille extérieure, le long de la partie médiane des deux bords longitudinaux opposés de cette feuille, la feuille intérieure étant fixée par collage à la feuille extérieure dans la zone de contact des feuilles autour du coussin, et des moyens d'attache pour fermer la couche-culotte autour du corps de l'utilisateur.

L'invention se rapporte également à un procédé de fabrication en continu de telles couches-culottes.

Le procédé usuel de fabrication en continu de couches-culottes connues du type défini ci-dessus consiste

- à dérouler une bande continue de feuille imperméable aux liquides,
- à appliquer des lignes longitudinales continues de colle sur la face supérieure de ladite bande,
- à dérouler des éléments élastiques continus, à encoller lesdits éléments élastiques par intervalles de manière à disposer de sections encollées séparées par des sections non encollées, et à les appliquer à l'état tendu sur la face supérieure de la bande imperméable entre les lignes de colle au voisinage des deux bords longitudinaux opposés de la bande, de manière à faire adhérer les éléments élastiques par sections successives espacées à la bande imperméable,
- à déposer successivement, sur la face supérieure de la bande imperméable munie des éléments élastiques, des coussins absorbants individuels de largeur inférieure à la largeur de la bande de manière que les coussins successifs soient disposés sur la bande dans les zones où les éléments élastiques adhèrent à cette dernière et soient espacés les uns des autres dans le sens de l longueur de la bande imperméable,
- à dérouler une bande continue de feuille perméable aux liquides ayant la même largeur que la bande imperméable, à encoller ladite bande sur une face, et à l'appliquer par ladite face encollée sur la face supérieure de la bande imperméable déjà munie des éléments élastiques et des coussins absorbants, et

- à découper successivement dans le sens transversal les deux bandes et les éléments élastiques tendus, entre le coussins successifs espacés, c'est-à-dire dans les sections non encollées des éléments élastiques.

Lors de ce découpage transversal qui fournit les couches-culottes individuelles, les éléments élastiques initialement continus et maintenus à l'état tendu sont sectionnés, de sorte que les parties des éléments élastiques disposées sur chaque couche-culotte de part et d'autre des sections encollées, adhérant à la feuille imperméable, se contractent, c'est-à-dire se détendent librement, alors que les sections encollées adhérant à la feuille imperméable se contractent avec ladite feuille pour donner à cette dernière, dans la zone d'entre-jambes de la couche-culotte, l'élasticité requise pour assurer une bonne adaptation au corps de l'utilisateur et une bonne étanchéité.

Toutefois, pour permettre une détente libre des parties non encollées des éléments élastiques lors de ce sectionnement, il est nécessaire de faire également en sorte que ces parties n'adhèrent pas à la feuille perméable encollée. C'est la raison pour laquelle on prévoit jusqu'à présent, dans l'encollage de cette feuille perméable, des zones non encollées à l'endroit des sections non encollées des éléments élastiques avant le sectionnement.

Cela donne donc naissance, sur la couche-culotte, dans la zone comprise entre les bords transversaux espacés du coussin absorbant d'une part, et des feuilles perméable et imperméable d'autre part, à des "tunnels" s'étendant entre ces deux feuilles depuis les bords transversaux du coussin jusqu'aux bords transversaux des feuilles, c'est-à-dire faisant communiquer avec l'extérieur l'espace compris entre les deux feuilles et contenant le coussin absorbant. Ces "tunnels" non seulement favorisent l'échappement cers l'extérieur, par effet de drainage, de l'urine absorbée par la coussin, mais permettent également l'échappement vers l'extérieur de la matière constitutive particulaire du coussin. Tel est le cas par exemple pour des coussins absorbant en pulpe de cellulose défibrée, mais en particulier pour des coussins contenant de la matière superabsorbante en grains qui, bien qu'étant généralement incorporée à une autre matière, par exemple à de la pulpe de cellulose défibrée, se détache souvent trés facilement du coussin absorbant et peut, en s'échappant par les "tunnels" en question, parvenir à l'extérieur et en raison de sa présentation sous forme de grains trés fins et légers à l'état sec, avoir ici des effect gênants, voire nuisibles pour les utilisateurs.

Il est par ailleurs connu par le document DE-A-33 19 043 de prévoir, sur une couche-culotte du type défini ci-dessus, deux rubans de feuille imperméables aux liquides, mais perméables à l'air, placés sur la face interne de la feuille extérieure, en dessous du coussin absorbant, sur toute la longueur de la feuille extérieure et sur une largeur supérieure à la largeur des éléments élastiques, les rubans étant fixés à la feuille extérieure par au moins une ligne longitudinale de colle de part de d'autre des éléments élastiques. Ces rubans sont destinés à empêcher des fuites de liquide à travers la feuille extéreure qui est poreuse en dessous des rubans, grâce au fronçage que les éléments élastiques provoquent sur la feuille extérieure et sur les rubans, empêchant ainsi leur contact réciproque et par conséquent un effet de mèche.

La présente invention a pour objet une couche-culotte du type défini ci-dessus sur laquelle le risque d'échappement vers l'extérieur de matière constiutive du coussin absorbant est empêché avec certitude et le risque d'échappement vers l'extérieur, tant a l'endroit des boris transversaux de la couche-culotte que sur les bords latéraux (longitudinaux) du coussin absorbant, des liquides absorbés par ce dernier, est empêché ou pour le moins fortement réduit.

l'invention a également pour objet un procédé de fabrication en continu de telles couches-culottes.

Le couche-culotte conforme à l'invention telle que reveniquée comprend une feuille extérieure souple, imperméable aux liquides, dont la face interne est munie de lignes longitudinales de colle. La couche-culotte comprend, en outre, un matelas absorbant disposé sur la face interne de la feuille extérieure de manière que ses deux bords longitudinaux opposés soient en retrait par rapport aux deux bords longitudinaux opposés de la feuille extérieure et ses deux bords transversaux opposés en retrait par rapport aux deux bords transversaux opposés de la feuille extérieure. La couche-culotte comprend, par ailleurs, une feuille intérieure souple, perméable aux liquides, recouvrant la face interne de la feuille extérieure et le matelas absorbant disposé sur certte face. Des moyens d'attache sont prévus pour fermer la couche-culotte autour du corps de l'utilisateur. Par ailleurs, des éléments élastiques longitudinaux sont fixés par collage à l'état tendu sur la face interne de la feuille extérieure le long de la partie médiane des deux bords longitudinaux de cette dernière, la feuille intérieure étant fixée par collage à la feuille extérieure, dans la zone de contact des deux feuilles autour du matelas. La couche-culotte comprend également deux rubans de feuille souple imperméables aux liquides placés sur la face interne de la feuille extérieure, en dessous du coussin absorbant, pardessus les éléments élastiques, sur toute la longueur de la feuille extérieure et sur une largeur supérieure à la largeur de ces éléments élastiques, de manière que les rubans soient fixés à la feuille extérieure par au moins une ligne longitudinale de colle de la feuille extérieure, de part et d'autre des éléments élastiques.

Selon l'invention, les rubans présentent une largeur et sont fixés sur la feuille extérieure de manière à dépasser le coussin sur toute sa longueur latéralement. les parties des rubans dépassant le coussin latéralement étant repliées sur le dessus du coussin de manière à envelopper les bords latéraux de ce dernier t et la face externe de la feuille intérieure est encollée sur tout le pourtour de manière à adhérer, tout autour du coussin, sur la feuille extérieure et sur les rubans aux endroits où lesdits rubans dépassent le coussin absorbant.

Les deux rubans s'étendant sur toute la longueur de la feuille extérieure empêchent tout contact de la feuille intérieure, bien qu'encollée sur tout le pourtour, avec les éléments élastiques contenus dans les "tunnels" que ces rubans forment avec la feuille extérieure, de sorte que les parties non encollées des éléments élastiques peuvent se contracter librement lors du sectionnement. De plus, la matière constitutive du coussin absorbant disposé au-dessus de ces rubans ne peut pas pénétrer dans lesdits tunnels et s'échapper vers l'extérieur, du fait que les tunnels ne sont ouverts qu'aux deux bords transversaux de la couche-culotte.

Le procédé conforme á l'invention de fabrication en continu de couches-culottes telles que définies ci-dessus consiste

- à dérouler une bande continue de feuille imperméable aux liquides,
- à appliquer en continu des lignes longitudinales de colle, espacées transversalement sur la face supérieure de ladite bande imperméable,
- à dérouler des éléments élastiques continus, à encoller lesdits éléments élastiques par intervalles pour former des sections encollées séparées par des sections non encollées, et à appliquer les éléments élastiques à l'état tendu sur la face supérieure de la bande imperméable, entre les lignes de colle longitudinales, au voisinage des deux bords longitudinaux opposés, de manière à y faire adhérer les éléments élastiques par sections successives espacées,
- à dérouler et à appliquer en continu sur la face supérieure de la bande imperméable, par dessus les éléments élastiques, deux rubans continus de feuille imperméables aux liquides ayant chacun une largeur supérieure à la largeur des éléments élastiques, de manière que chaque ruban dépasse lateralement vers l'extérieur le bord longitudinal correspondant de de la bande imperméable et adhère à la bande imperméable par au moins une ligne longitudinale de colle de part et d'autre des éléments élastiques,
- à déposer successivement sur la face supérieure de la bande imperméable, par-dessus les rubans, des coussins absorbant individuels de largeur inférieure à la largeur de la bande de manière que les coussins successifs soient disposés sur la bande imperméable dans les zones où les éléments élastiques adhèrent à la bande, et soient espacés les uns des autres dans le sens de la longueur de la bande imperméable,
- à replier, surle dessus des coussins, les parties des rubans dépassant latéralement les coussins,
- à dérouler une bande continue perméable aux liquides, ayant sensiblement la même largeur que la bande imperméable, à encoller ladite bande perméable sur une face et à l'appliquer par ladite face encollée sur la face supérieure de la bande imperméable, pardessus les rubans et les coussins absorbants, et
- à découper successivement, dans le sens transversal, les deux bandes, les éléments élastiques tendus et les deux rubans, entre les coussins successifs espacés, à l'endroit de sections non encollées des éléments élastiques.

Bien qu'il soit possible d'encoller la bande perméable aux liquides sur l'ensemble de sa première face, il est également possible d'encoller cette feuille sur sa première face suivant un dessin répétitif laissant subsister, au milieu de la largeur de la bande, des fenêtres non encollées successives qui sont entourées par des cadres complets encollés et qui, lors de l'application de la feuille perméable sur la feuille imperméable, viennent se placer sur les coussins absorbant disposés sur la feuille perméable, alors que les cadres encollés viennent adhérer à la feuille inperméable et aux rubans fixés à cette dernière, sur tout le pour tour du coussin absorbant.

En se référant aux dessins schématiques annexés, on va décrire ci-après plus endétail un mode de réalisation illustratif et non limitatif d'une couche-culotte conforme à l'invention, et un procédé de fabrication en continu de telles couches-culottes; sur les dessins:

la figure 1 est une vue sur la face interne d'une couche-culotte représentée à plat, les éléments élastiques étant tendus, avec deux arrachements partiels montrant la structure interne de la couche-culotte;

la figure 2 est une coupe, à plus grande échelle, suivant II-II de la figure 1;

la figure 3 est une coupe, à plus grande échelle, suivant III-III de la figure 1;

la figure 4 montre les différentes étapes d'un procédé de fabrication en continu de couches-culottes suivant les figures 1 à 3;

les figures 5 à 7 représentent, de façon analogue aux figures 1 à 3, un mode de réalisation d'une couche-culotte conforme à l'invention.

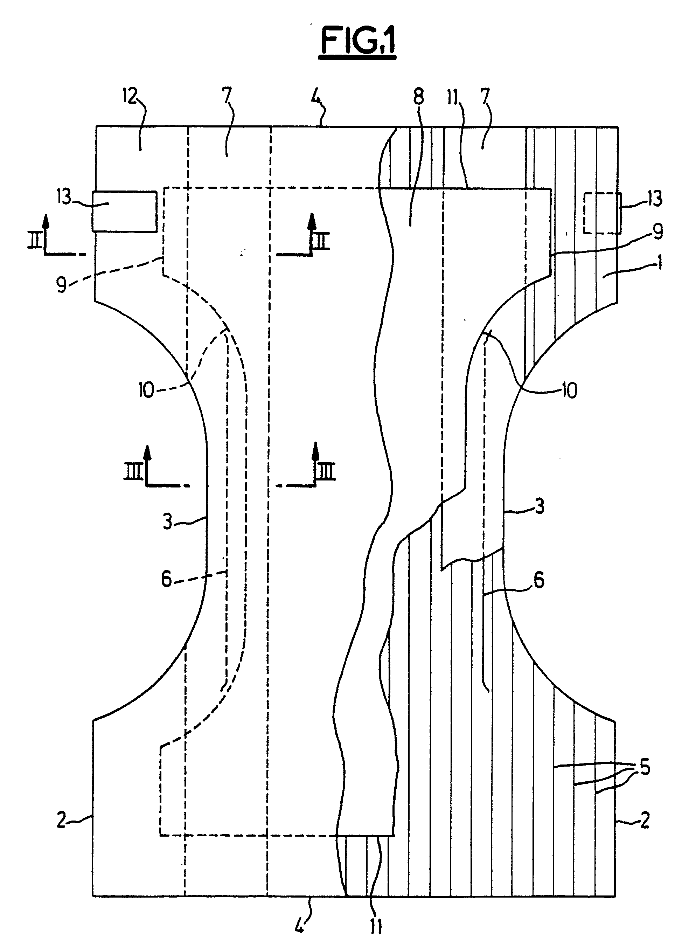

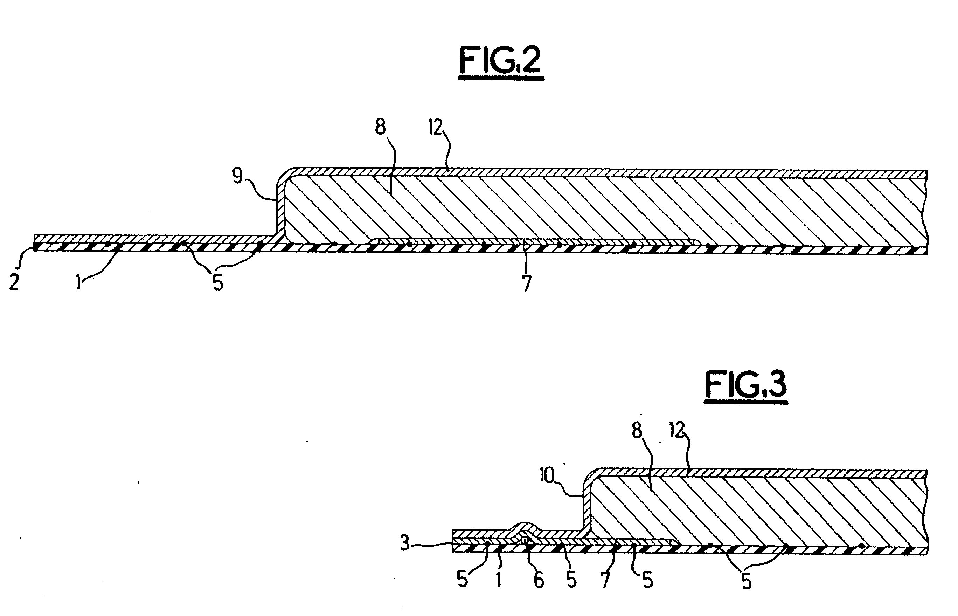

La couche-culotte telle qu'illustrée par les figures 1, 2 et 3 comprend une feuille extérieure 1 souple, imperméable aux liquides, par exemple une feuille de matière plastique telle que de polyéthylène, présentant une forme généralement rectangulaire avec deux bords longitudinaux 2 opposés munis chacun d'une échancrure 3 médiane, et deux bords transversaux 4 opposés, rectilignes. La feuille 1 est munie sur sa face intérieure, apparente sur la figure 1, d'une multitude de lignes de colle 5 longitudinales, espacées.

Un élément élastique 6 longitudinal est fixé à l'état tendu, par collage, sur la face intérieure de la feuille 1, sur la partie médiane de la longueur de la feuille 1, le long de chaque échancrure 3, avec un léger décalage vers l'intérieur par rapport au fond de l'échancrure.

Un ruban 7 d'un matériau souple en feuille, par exemple du papier enduit ou non ou une feuille de matière plastique, est fixé sur toute la longueur de la feuille I, le long de chacun des deux côtés longitudinaux de cette hernière, de manière à recouvrir chacun l'un des éléments élastiques 6. Il y a lieu de noter que la largeur des rubans 7 est telle que chaque ruban 7 adhère à la feuille I suivant au moins une ligne de colle 5 de part et d'autre de l'élément élastique 6 correspondant. Chaque ruban 7 forme donc, avec la feuille I, un tunnel longitudinal contenant l'élément élastique et s'étendant sur toute la longueur de la feuille 1, ce tunnel étant délimité par le ruban 7, la feuille 1 et deux lignes de colle 5 de part et d'autre de l'élément élastique 6.

Un coussin absorbant 8, également de forme générale rectangulaire, avec deux bords longitudinaux 9 opposés munis chacun d'une échancrure médiane 10, et deux bords transversaux 11 opposés rectilignes, est fixé sur la face intérieure de la feuille 1, par dessus les rubans 7, de manière que ses bords longtiudinaux 9 et transversaux 11 se trouvent en retrait par rapport aux bords longitudinaux 2 et transversaux 4 de la feuille 1. Le coussin absorbant 8 peut être constitué par exemple de pulpe de cellulose défibrée, contenant ou non de la matière superabsorbante en grains. La fixation de coussin 8 sur la feuille I s'effectue par les lignes de colle 5 qui restent apparentes après la mise en place des rubans 7.

L'ensemble de la feuille 1 est recouverte, sur la face intérieure portant les rubans 7 et le coussin absorbant 8, d'un voile 12 perméable aux liquides, par exemple un voile de non-tissé. Ce voile 12 est muni, sur sa face tournée vers la feuille 1, d'un encollage tel que la feuille 12 adhère au moins à la feuille 1 et aux rubans 7 dans les zones où le feuille 1 est recouverte par les rubans 7, sur tout le pourtour du coussin absorbant 8, c'est-à-dire entre les bords 9, 10, 11 du coussin 8 et les bords 2, 3, 4 de feuille 1.

Cet encollage intéresse soit toute la surface de la feuille 12, auquel cas cette dernière adhère également au coussin absorbant 8, ou alors est réalisé selon un motif en forme de cadre fermé entourant une fenêtre, qui peut par exemple avoir une forme rectangulaire ou la forme du coussin absorbant 8.

Enfin, des attaches adhésives 13 de type connu sont fixées sur les deux bords longitudinaux 2 opposés de la couche-culotte ainsi constituée, au voisinage de l'un des bords transversaux 4, c'est-à-dire d'un côté des deux échancrures 3 opposées, pour permettre la fermeture de la couche-culotte autour de la taille de l'utilisateur.

Il apparaît sur les figures 1, 2 et 3 que chaque ruban 7 définit avec la feuille 1, pour chaque élément élastique 6, sur toute la longueur de la couche-culotte, un tunnel délimité de part et d'autre de l'élément élastique 6 par au moins une ligne de colle 5. Par conséquent, ces tunnels s'étendant sur toute la longueur de la couche-culotte et débouchant vers l'extérieur aux deux extrémités transversales 4 de la couche-culotte sont complètement isolés du coussin absorbant 8 disposé sur les rubans 7. Dans ces conditions, la matière constitutives du coussin absorbant 8 ne peut en aucune manière pénétrer dans le tunnel défini par les ruban 7 et s'échapper de là vers l'extérieur.

Les rubans 7 étant constitués par une matière imperméable aux liquides, par exemple du polyéthylène ou du papier enduit, ces tunnels ne sont pas non plus accessibles aux liquides absorbés pas le coussin 8, empêchant ainsi tout échappement des liquides (urine) par effet de drainage par ledit tunnel, aux deux extrémités transversales de la couche-culotte, extrémités qui, sur la couche-culotte fermée, correspondent à la ceinture de la couche-culotte.

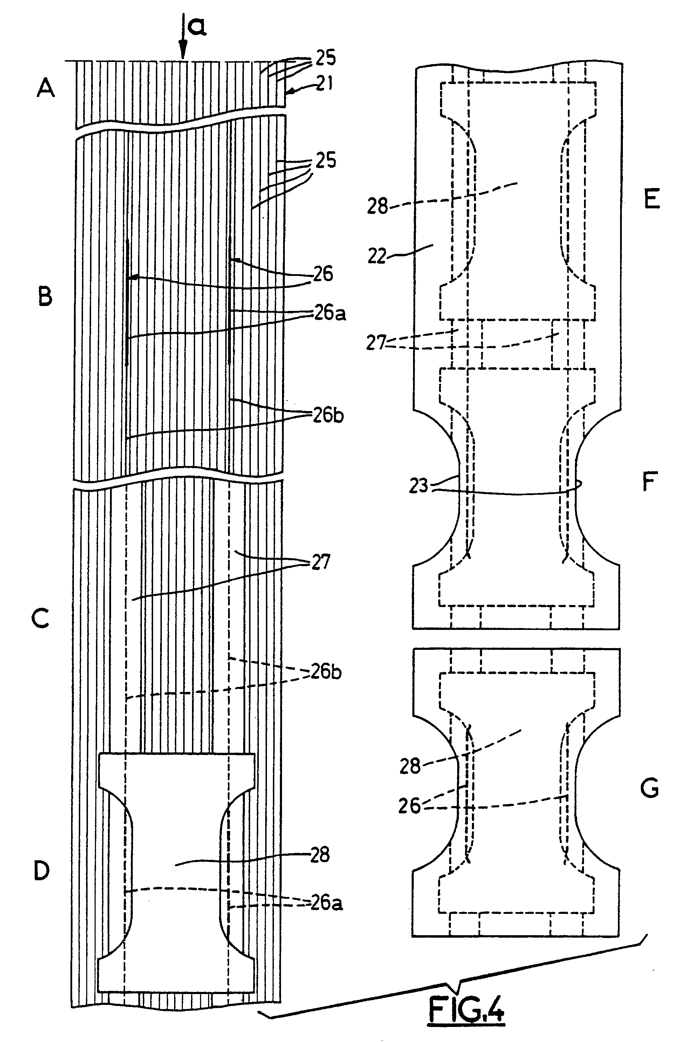

On va maintenant décrire, en se référant à la figure 4, un procédé pour la fabrication en continu de couches-culottes suivant les figures 1 à 3.

Pour fabriquer en continu les couches-culottes, on déroule d'abord en continu une bande 21 de feuille imperméable aux liquides, d'une largeur correspondant à la largeur des couches-culottes à fabriquer. Sur la face supérieure de la bande 21 ainsi déroulée, avançant dans le sens de la flèche a, on applique une multitude de lignes de colle 25 longitudinales espacées sur toute la largeur de la bande 21 (phase A).

On déroule ensuite en continu deux éléments élastiques 26 continus, en encolle lesdits éléments élastiques 26 par intervalles de manière à former des sections encollées 26a séparées par des sections 26b non encollées, et on applique les deux éléments élastiques à l'état tendu sur la face supérieure de la bande 21, entre les lignes de colle 25, en retrait vers l'intérieur par rapport aux deux bords longitudinaux opposés de la bande, de manière à faire adhérer les éléments élastiques 26 à l'état tendu par les sections encollées 26a à la bande (phase B).

On déroule ensuite en continu deux rubans 27 d'une matière en feuille souple, imperméable aux liquides, et on les applique en continu sur la face supérieure de la bande 21, par-dessus les deux éléments élastiques 26, la largeur des rubans 27 étant telle que chaque ruban 27 soit relié par au moins une ligne de colle 25 à la bande 21 de part et d'autre de chaque élément élastique 26 (phase C).

On dépose alors, sur la face supérieure de la bande 21 garnie des élastiques 26 et des rubans 27, successivement, des coussins absorbants 28 en forme de sablier, de largeur inférieure à la largeur de la bande 21, chaque coussin 28 étant disposé symétriquement par rapport à une section encollée 26a des éléments élastiques 26 et symétriquement par rapport aux deux bords longitudinaux de la bande 21 (phase D).

Ensuite, on déroule une bande continue 22 de feuille souple perméable aux liquides, ayant la même largeur que la bande 21, on encolle la bande 22 sur une face et on l'applique par ladite face encollée sur la face supérieure de la bande 21, par dessus les coussins 28, de manière que la bande 32 adhère, tout autour des coussins 28, à la bande 21 et aux parties des rubans 27 non recouvertes par les coussins 28 (phase E).

On découpe par la suite dans les deux bandes 21 et 22, au milieu de la longueur de chaque coussin 28, deux échancrures latérales opposées 23 dans les deux bords longitudinaux des bandes 21 et 32 (phase F).

Après avoir ensuite fixé, sur les deux bords longitudinaux des bandes 21, 22, à l'endroit d'une partie plus large de chaque coussin 28, des attaches adhésives non représentées sur la figure 4, on découpe dans le sens transversal les deux bandes 21 et 32 réunies, les éléments élastiques 26 tendus et les rubans 27 entre les coussins successifs 28, c'est-à-dire dans les sections non encollées 26b des éléments élastiques 26 (phases F et G).

Le sectionnement, à l'endroit des sections 26b non encollées, des éléments élastiques 26 maintenus jusqu'à présent à l'état tendu a pour effet libérer les éléments élastiques 26 de leur tension initiale, de sorte que les sections 26b non encollées se détendent en se contractant librement, alors que les section 26a encollés adhèrent à la fois à la feuille 21 et aux rubans 27 et ne peuvent donc se contracter que conjointement avec la feuille 21 et les rubans 27. On reconnaît sur la figure 4 (phase G) cette contraction libre des parties non encollées des éléments élastiques, alors que les parties encollées des éléments élastiques sont encore tendues du fait que la couche-culotte obtenue par sectionnement de la bande composite est encore maintenue à l'état allongé par des moyens non représentés. Après libération de la couche-culotte, les parties encollées des éléments élastiques se détendent en contractant élastiquement la couche-culotte dans le sens de la longueur dans la zone de d'entre-jambes, de largeur réduite.

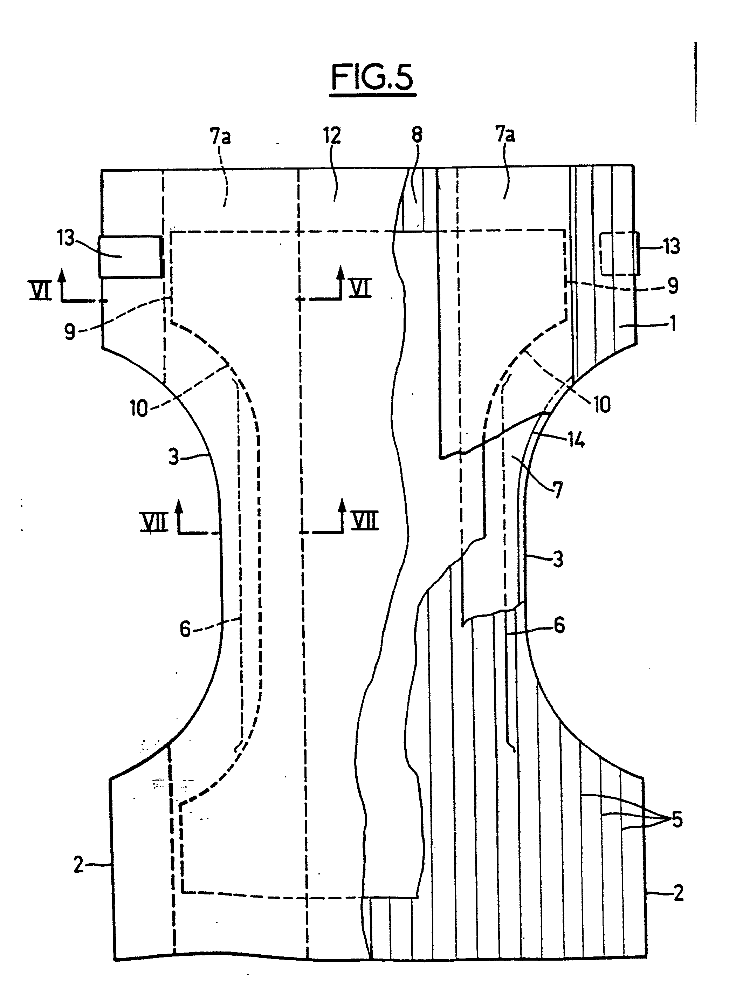

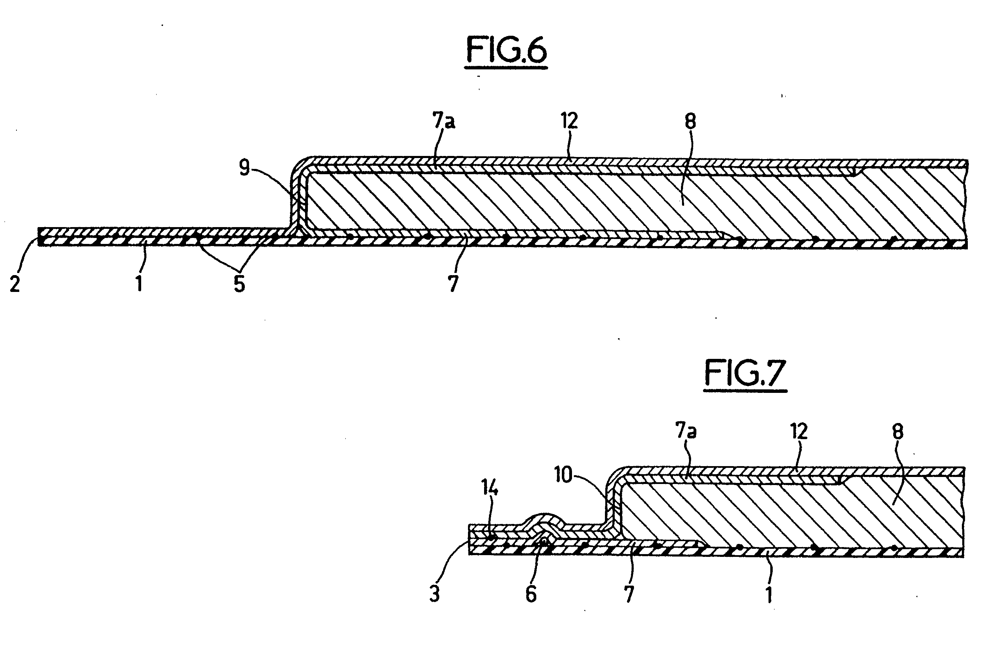

La couche-culotte conforme à l'invention suivant les figures 5 à 7 diffère de celle du mode de réalisation des figures 1 à 3 par le fait que les rubans 7 sont plus larges de manière à dépasser le coussin 8 latéralement, et que les parties 7a des rubans dépassant le coussin latéralement sont repliées sur le dessus de coussin 8. Ces parties 7a des rubans enveloppent ainsi les bords latéraux du coussin sur toute la longueur, c'est-à-dire à l'endroit des échancrures médianes 10 et de part et d'autre des échancrures médianes (zones 9). Dans la zone de la feuille 1 et du voile 12, où les parties dépassantes 7a des rubans 7 subissent une découpe à l'endroit des échancrures 3, les deux plis superposés des rubans 7 sont reliés entre eux par une ligne de soudure 14 produite par le découpage par fusion des échancrures, ou par une ligne de colle appliquée préalablement au repliage des parties 7a. Les rubans, 7, 7a enveloppent ainsi les bords latéraux du coussin sur toute la longueur, empêchant ainsi tout échappement de liquide du coussin 8 sur ces bords. De plus, les parties repliées 7a des rubans 7 réduisant la surface du coussin 8 en contact avec la peau de l'utilisateur par l'intermédiaire du voile 12.

La fabrication de la couche-culotte conforme à invention suivant les figures 5 à 7 ne diffère de celle de la couche-culotte suivant les figures 1 à 3, décrite avec référence à la figure 4, que par l'utilisation de rubans 27 plus larges, par une opération de repliage, sur le dessus des coussins 28, des parties des rubans 27 dépassant latéralement les coussins 28 (entre les étapes C et D), et par la prévision éventuelle de lignes de collage sur les rubans, avant le repliage (lignes 14).

L'avantage particulier d'une telle couche-culotte conforme à l'invention, par rapport à une couche-culotte suivant la demande de brevet français n° 2 557 774 sur laquelle des rabats entourant les bords latéraux du coussin sont constitués par la matière de la feuille extérieure de la couche-culotte, consiste dans le fait que les rabats sont constitués par une feuille ayant uniquement une fonction d'étanchéité et pouvant donc avoir une épaisseur nettement plus faible qu'une feuille extérieure ayant également une fonction de support (par exemple environ 10 $\mu$m au lieu d'environ 20 à 30 $\mu$m pour une feuille extérieure) d'où une réduction du prix de revient de la couche-culotte.

Il va de soi que la couche-culotte conforme à l'invention telle que illustrée par les figures 5 à 7, et le procédé de fabrication en continu de telles couches-culottes n'ont été décrits qu'à titre d'exemple illustratif et non limitatif et que de nombreuses modifications et variantes sont possibles dans le cadre de l'invention.

Ainsi, l'invention est applicable non seulement à des couches-culottes en forme de sablier, mais également à des couches-culottes rectangulaires.

Au lieu de comporter un seul élément élastique sur chaque côté longitudinal du coussin absorbant, la couche-culotte pourrait également comporter des groupes de plusieurs éléments élastiques parallèles, par exemple au nombre de deux ou de quatre, chaque groupe d'éléments élastiques étant recouvert par un même ruban.

La feuille extérieure de la couche-culotte peut être constituée de toute matière en feuille souple, imperméable aux liquides et imperméable ou de préférence perméable à l'air, par exemple une feuille de polyéthylène, le cas échéant rendue perméable à l'air.

Le coussin absorbant peut être de nature quelconque, mais l'invention est particulièrement intéressante dans le cas de couches-culottes comprenant des coussins absorbants renfermant de la matière particulaire, notamment de la matière superabsorbante en grains qui, sur les couches-culottes connues, risque de s'échapper, alors que ce risque est complètement éliminé sur la couche-culotte conforme à l'invention, grâce à la présence des rubans 7, 27.

## Revendications

1.    Couche-culotte du type comprenant une feuille extérieure (1) souple, imperméable aux liquides dont la face interne est munie de lignes longitudinales de colle (5), un coussin absorbant (8) disposé sur la face interne de la feuille extérieure de manière que ses deux bords longitudinaux opposés (9) soient en retrait par rapport aux deux bords longitudinaux opposés (2, 3) de la feuille extérieure et que ses deux bords transversaux opposés (11) soient en retrait par rapport aux deux bords transversaux opposés (4) de la feuille extérieure, une feuille intérieure souple (12), perméable aux liquides, recouvrant la face interne de la feuille extérieure et le coussin absorbant disposé sur cette face, des éléments élastiques longitudinaux (6) fixés par collage à l'état tendu sur la face interne de la feuille extérieure, le long de la partie médiane des deux bords longitudinaux opposés de cette feuille, la feuille intérieure étant fixée par collage à la feuille extérieure dans la zone de contact des feuilles autour du coussin, des moyens d'attache (13) pour fermer la couche-culotte autour du corps de l'utilisateur, et deux rubans de feuille souple (7) imperméables aux liquides, placés sur la face interne de la feuille extérieure, en dessous du coussin absorbant (8), par dessus les éléments élastiques (6), sur toute la longueur de la feuille extérieure et sur une largeur supérieure à la largeur des éléments élastiques, les rubans étant fixés à la feuille extérieure par au moins une ligne longitudinale de colle de part et d'autre des éléments élastiques, caractérisée par le fait que les rubans (7) présentent une largeur et sont fixés sur la feuille extérieure de manière à dépasser le coussin (8) sur toute sa longueur latéralement, les parties (7a) des rubans dépassant le coussin (8) latéralement étant repliées sur le dessus du coussin de manière à envelopper les bords latéraux de ce dernier, et que la face externe de la feuille intérieure est encollée sur tout le pourtour de manière à adhérer, tout autour du coussin (8), sur la feuille extérieure (1) et sur les rubans (7) aux endroits où lesdits rubans dépassent le coussin absorbant.

2. Couche-culotte suivait la revendication 1, caractérisée par le fait qu'elle comprend un coussin absorbant contenant de la matière sous forme particulaire, en particulier de la matière superabsorbante en grains.

3. Procédé de fabrication en continu de couches-culottes suivant la revendication 1 ou 2, caractérisé par le fait qu'il consiste:
   - à dérouler une bande continue de feuille imperméable aux liquides,
   - à appliquer en continu des lignes longitudinales de colle espacées transversalement sur la face supérieure de ladite bande imperméable,
   - à dérouler des éléments élastiques continus, à encoller lesdits éléments élastiques par intervalles pour former des sections encollées séparées par des sections non encollées, et à appliquer les éléments élastiques à l'état tendu sur la face supérieure de la bande imperméable, au voisinage des deux bords longitudinaux opposés de cette dernière, de manière à y faire adhérer les éléments élastique par sections successives espacées,
   - à dérouler et appliquer en continu sur la face supérieure de la bande imperméable, par dessus les éléments élastiques, deux rubans continus de feuille imperméables aux liquides ayant chacun une largeur supérieure á la largeur des éléments élastiques de manière que chaque ruban dépasse latéralement vers l'extérieur le bord longitudinal correspondant de la bande imperméable et adhère à la bande imperméable par au moins une ligne longitudinale de colle de part et d'autre des éléments élastiques,
   - à déposer successivement sur la face supérieure de la bande imperméable, par dessus les deux rubans, des coussins absorbants individuels de largeur inférieure à la largeur de la bande, de manière que les coussins successifs soient disposés sur la bande imperméable dans les zones où les éléments élastiques adhèrent à la bande et soient espacés les uns des autres dans le sens de la longueur de la bande imperméable, à replier, sur le dessus des coussins, les parties des rubans dépassant latéralement les coussins,
   - à dérouler une bande continue perméable aux liquides, ayant sensiblement la même largeur que la bande imperméable, à encoller ladite bande perméable sur une face et à l'appliquer par ladite face encollée sur la face supérieure de la bande imperméable, par-dessus les rubans et les coussins absorbants, de manière que la bande perméable adhère, tout autour des coussins absorbants, à la bande imperméable et aux rubans à l'endroit où lesdits rubans débordent sur les coussins absorbants, et
   - à découper successivement, dans le sens transversal, les deux bandes, les éléments élastiques tendus et les deux rubans, entre les coussins successifs espacés, à l'endroit des sections non encolpées des éléments élastiques.

4. Procédé suivant la revendication 3 caractérisé par le fait qu'on encolle la bande perméable suivant un motif répétitif en forme de cadre fermé entourant une fenêtre, chaque fenêtre non encollée venant se placer sur un coussin absorbant et le cadre encollé adhérant à la bande imperméable et aux rubans autour du coussin absorbant.

## Claims

1. Pair of nappy-pants, of the type comprising a liquid-impermeable, flexible outer sheet (1) whose internal face is provided with longitudinal lines of glue (5), an absorbent pad (8) arranged on the internal face of the outer sheet such that its two opposite longitudinal edges (9) are set back relative to the two opposite longitudinal edges (2, 3) of the outer sheet, and such that its two opposite transverse edges (11) are set back relative to the two opposite transverse edges (4) of the outer sheet, a liquid-permeable, flexible inner sheet (12), covering the internal face of the outer sheet and the absorbent pad arranged on this face, longitudinal elastic elements (6) fastened by adhesive bonding in the taut state onto the internal face of the outer sheet, along the mid part of the two opposite longitudinal edges of this sheet, the inner sheet being fastened by adhesive bonding to the outer sheet in the zone of contact of the sheets around the pad, attachment means (13) for closing the pair of nappy-pants around the body of the user, and two liquid-impermeable tapes (7) of flexible sheet placed on the internal face of the outer sheet, beneath the absorbent pad (8) and on top of the elastic elements (6), over the entire length of the outer sheet and over a width greater than the width of the elastic elements, the tapes being fastened to the outer sheet by at least one longitudinal line of glue on either side of the elastic elements, characterized in that the tapes (7) have a width and are fastened onto the outer sheet such that they protrude from the pad (8) over its entire length laterally, those parts (7a) of the tapes protruding from the pad (8) laterally being folded back on top of the pad so as to envelop the lateral edges of the latter, and in that the external face of the inner sheet is glued over its entire periphery so as to adhere all around the pad (8), to the outer sheet (1) and to the tapes (7) at the locations where the said tapes protrude from the absorbent pad.

2. Pair of nappy-pants according to Claim 1, characterized in that it comprises an absorbent pad containing particulate material, in particular superabsorbent granular material.

3. Method for the continuous manufacture of nappy-pants according to Claim 1 or 2, characterized in that it consists:
   - in unwinding a continuous strip of liquid-impermeable sheet,
   - in continuously applying longitudinal lines of glue, which lines are spaced apart transversely on the upper face of the said impermeable strip,
   - in unwinding continuous elastic elements, in glueing the said elastic elements at intervals in order to form glued sections separated by non-glued sections, and in applying the elastic elements in the taut state to the upper face of the impermeable strip, in the vicinity of the two opposite longitudinal edges of the latter, so as to cause the elastic elements to adhere thereto in spaced-apart successive sections,
   - in unwinding and continuously applying to the upper face of the impermeable strip, above the elastic elements, two continuous liquid-impermeable tapes of sheet, each having a width greater than the width of the elastic elements such that each tape protrudes laterally outwards from the corresponding longitudinal edge of the impermeable strip and adheres to the impermeable strip by at least one longitudinal line of glue on either side of the elastic elements,
   - in successively depositing on the upper face of the impermeable strip, on top of the two tapes, individual absorbent pads of width less than the width of the strip, such that the successive pads are arranged on the impermeable strip in the zones where the elastic elements adhere to the strip and are spaced apart from one another in the direction of the length of the impermeable strip,
   - in folding those parts of the tapes protruding laterally from the pads back onto the top of the pads,
   - in unwinding a liquid-permeable continuous strip having substantially the same width as the impermeable strip, in glueing the said permeable strip on one face and in applying it by the said glued face to the upper face of the impermeable strip, on top of the tapes and the absorbent pads, such that the permeable strip adheres, all around the absorbent pads, to the impermeable strip and to the tapes at the location where the said tapes protrude beyond the absorbent pads, and

- in cutting successively, in the transverse direction, the two strips, the taut elastic elements and the two tapes, between the spaced-apart successive pads, at the location of the non-glued sections of the elastic elements.

4. Method according to Claim 3, characterized in that the permeable strip is glued in a repeated pattern in the form of a closed frame surrounding a window, each non-glued window being placed on an absorbent pad and the glued frame adhering to the impermeable strip and to the tapes around the absorbent pad.

## Ansprüche

1. Windelhöschen, der Art mit einer weichen, für Flüssigkeiten undurchlässigen Außenlage (1) deren Innenseite mit längslaufenden Klebstofflinien (5) ausgestattet ist,mit einem saugfähigen Kissen (8) , das an der Innenseite der Außenlage so angeordnet ist, daß seine beiden gegenüberliegenden Längsränder (9) zu den beiden gegenüberliegenden Längsrändern (2,3) der Außenlage rückversetzt sind, und seine beiden gegenüberliegenden Querränder (11) zu den beiden gegenüberliegenden Querrändern (4) der Außenlage rückversetzt sind, mit eine weichen, für Flüssigkeiten durchlässigen Innenlage (12), welche die Innenseite der Außenlage und das auf dieser Seite angeordnete, saugfähige Kissen abdeckt, mit längslaufenden, elastischen Elementen (6), die durch Kleben im gespannten Zustand an der Innenseite der Außenlage, längs dem mittleren Teil der beiden gegenüberliegenden Längsränder dieser Lage befestigt sind, wobei die Innenlage durch Kleben an der Außenlage in dem Bereich, in dem die Lagen um das Kissen herum aneinander anliegen, befestigt ist, mit Verschlußmitteln (13) zum Schließen des Windelhöschens um den Körper des Benutzers und mit zwei weichen Streifen (7), aus für Flüssigkeiten undurchlässiger Folie, die auf der Innenseite der Außenlage unter dem saugfähigen Kissen (8), über den elastischen Elementen (6) auf der gesamten Länge der Außenfolie und auf einer Breite angeordnet sind, die größer ist als die Breite der elastischen Elemente, wobei die Streifen an der Außenlage durch wenigstens eine längslaufende Klebstofflinie beiderseits der elastischen Elemente befestigt sind, dadurch gekennzeichnet, daß die Streifen (7) eine solche Breite aufweisen und so an der Außenlage befestigt sind, daß sie seitlich über das Kissen (8) auf dessen gesamter Länge vorstehen, wobei die Teile (7a) der Streifen, die seitlich über das Kissen (8) vorstehen, auf die Oberseite des Kissens so umgeschlagen sind, daß sie die seitlichen Ränder des letzteren einhüllen, und daß die Außenseite der Innenlage auf dem ganzen Umfang mit Klebstoff so beschichtet ist, daß sie zur Gänze um das Kissen (8) herum auf der Außenlage (1) und auf den Streifen (7) in Bereichen anklebt, in welchen diese Streifen über das saugfähige Kissen vorstehen.

2. Windelhöschen nach Anspruch 1, dadurch gekennzeichnet, daß es ein saugfähiges Kissen aufweist, das einen teilchenförmigen Stoff enthält, insbesondere aus einem stark saugendem granulierten Werkstoff.

3. Verfahren zum kontinuierlichen Herstellen von Windelhöschen nach Anspruch 1 oder 2, dadurch gekennzeichnet, daß es umfaßt:

- Abrollen eines kontinuierlichen Bandes aus einer für Flüssigkeiten undurchlässigen Lage,
- kontinuierliches Aufbringen von längslaufenden Linien aus Klebstoff, auf die Oberseite des undurchlässigen Bandes, die quer von einander beabstandet sind,
- Abrollen von endlosen, elastischen Elementen, Umhüllen dieser elastischen Elemente mit Klebstoff in Intervallen, um klebstoffbeschichtete Abschnitte zu bilden, die voneinander durch nicht mit Klebstoff beschichtete Abschnitte getrennt sind, und Aufbringen der elastischen Elemente im gespannten Zustand auf die Oberseite des undurchlässigen Bandes in der Nähe der beiden gegenüberliegenden Längsränder des letzteren, so daß dort die elastischen Elemente in aufeinanderfolgenden, beabstandeten Abschnitten ankleben,
- Abrollen und kontinuierliches Aufbringen von zwei kontinuierlichen Streifen aus für Flüssigkeiten undurchlässiger Folie, die jeweils eine Breite haben, die größer ist als die Breite der elastischen Elemente, auf die Oberseite des undurchlässigen Bandes, oberhalb der elastischen Elemente, derart, daß jeder Streifen den entsprechenden Längsrand des undurchlässigen Bandes seitlich nach außen überragt und am undurchlässigen Band durch wenigstens eine längslaufende Klebstofflinie beiderseits der elastischen Elemente anhaftet,

- nacheinander Ablegen saugfähiger, einzelner Kissen mit einer Breite, die kleiner ist, als die Breite des Bandes, auf die Oberseite des undurchlässigen Bandes, oberhalb der beiden Streifen, derart, daß die aufeinanderfolgenden Kissen auf dem undurchlässigen Band in den Bereichen angeordnet sind, in welchen die elastischen Elemente am Band anhaften, und in der Längsrichtung des undurchlässigen Bandes voneinander beabstandet sind

- Umfalten der Teile der Streifen, welche die Kissen seitlich überragen, auf die Oberseite der Kissen,

- Abrollen eines endlosen, für Flüssigkeiten durchlässigen Bandes, das im wesentlichen die gleiche Breite hat wie das undurchlässige Band, Beschichten des durchlässigen Bandes mit Klebstoff auf einer Seite und Aufbringen desselben, mit der mit Klebstoff beschichtete Seite auf die Oberseite des undurchlässigen Bandes, oberhalb der Bänder und saugfähigen Kissen, so daß das durchlässige Band zur Gänze um die saugfähigen Kissen herum am undurchlässigen Band und an den Bändern, in dem Bereich, wo diese Streifen über die saugfähigen Kissen vorstehen, anhaftet, und

- nacheinander Abschneiden der beiden Bänder, der gespannten, elastischen Elemente und der beiden Streifen in Querrichtung, zwischen den voneinander beabstandeten, aufeinanderfolgenden Kissen im Bereich der nicht mit Klebstoff beschichteten Abschnitte der elastischen Elemente.

4. Verfahren nach Anspruch 3, dadurch gekennzeichnet, daß man das durchlässige Band, gemäß einem sich wiederholenden Musters in Form eines geschlossenen Rahmens, der ein Fenster umgibt, mit Klebstoff beschichtet, wobei jedes nicht mit Klebstoff beschichtete Fenster auf einem saugfähigen Kissen angelegt wird, und der mit Klebstoff beschichtete Rahmen um das saugfähige Kissen herum am undurchlässigen Band und an den Streifen haftet.

# FIG.1

FIG.2

FIG.3

EP 0 270 979 B1

FIG.4

# FIG.5

## FIG.6

## FIG.7

EP 0 270 979 B1